# EUROPEAN PATENT SPECIFICATION

(11) **EP 3 325 644 B1**
(45) Date of publication and mention of the grant of the patent: **09.03.2022**
(21) Application number: 16739154.9
(22) Date of filing: 15.07.2016
(51) Int. Cl.: C12Q 1/68

(54) **METHOD OF DETERMINING THE LEVEL OF DNA-CONTAINING CELLS IN A BIOLOGICAL SAMPLE AND MICROFLUIDIC DEVICES FOR THE IMPLEMENTATION OF THE METHOD**
VERFAHREN ZUR BESTIMMUNG DES NIVEAUS DNS-HALTIGER ZELLEN IN EINER BIOLOGISCHEN PROBE UND MIKROFLUIDISCHE VORRICHTUNGEN ZUR DURCHFÜHRUNG DES VERFAHRENS
PROCÉDÉ DE DÉTERMINATION DU NIVEAU DE CELLULES CONTENANT DE L'ADN DANS UN ÉCHANTILLON BIOLOGIQUE ET DISPOSITIFS MICROFLUIDIQUES POUR LA MISE EN OEUVRE DU PROCÉDÉ

(30) Priority: 17.07.2015 EP 15177313
(43) Date of publication of application: 30.05.2018
(73) Proprietor: Fluimedix APS, 2600 Glostrup (DK)
(72) Inventor: USSING, Tomas, 2000 Frederiksberg (DK); SKOVMOLLER, Marie, 5000 Odense (DK)
(74) Representative: Guardian IP Consulting I/S
(86) International application number: PCT/EP2016/066927
(87) International publication number: WO 2017/013024

(56) References cited:
- EP-A1- 2 755 025
- WO-A1-01/35728
- WO-A1-2007/075287
- WO-A1-2010/079469
- WO-A2-2011/119492
- VIGUIER C ET AL: "Mastitis detection: current trends and future perspectives", TRENDS IN BIOTECHNOLOGY, ELSEVIER PUBLICATIONS, CAMBRIDGE, GB, vol. 27, no. 8, 1 August 2009 (2009-08-01) , pages 486-493, XP026349419, ISSN: 0167-7799 [retrieved on 2009-07-16]

## Description

### FIELD OF THE INVENTION

The present invention pertains to a method of determining the level of DNA-containing cells in a biological sample and devices for the implementation of the method. The present invention is particularly useful for quantification of somatic cells in milk samples, which is used as an indicator for the presence of mastitis in the udder of the cow that has produced the milk.

### BACKGROUND

Mastitis tests are performed routinely by the farmer on the milk of individual cows in order to monitor the health condition of the cows and to avoid that the milk of a single infected udder quarter fouls the pooled milk collected from the cattle herd.

Conventionally cow-side testing has been performed using the California Mastitis Test (CMT) which is an indicator of the somatic cell count of milk. The test operates by disrupting the cell membrane of any cells present in the milk sample, allowing the DNA in those cells to react with the test reagent, forming a gel. The California Mastitis Test is e.g. described by Barnum et al, (Barnum et al; The Canadian Veterinary Journal; vol 2; no 3; March 1961; p 83-90).

Quantification of DNA containing somatic cells is also traditionally used for determining the level of white blood cells in blood samples. A high level of white blood cells is used as an indicator of infection.

WO 01/35728 A1 describes testing procedures for milking animals whereby a milk flow has a sample flow diverted therefrom which is then tested for somatic cell count indicative of mastitis. Such testing of the diverted flow is automatic and involves a measure of viscosity change in the sample after the addition of an anionic detergent which causes gelling to an extent determined by the somatic cell count (similar to the California Mastitis Test). As a consequence calibratable data from each milking animal can be generated and used as a comparative tool between animals and/or to monitor changes in the somatic cell count (SCC) of individual animals between milkings.

However, WO 01/35728 A1 neither discloses nor suggests the implementation of the test in a microfluidic device and it is silent about the challenges and benefits such implementation gives rise to.

WO 2010/079469 A1 describes a method of estimating the somatic cell count for a liquid, typically milk. The method involves mixing a sample of milk with a detergent (similar to the California Mastitis Test), and then allowing the mixture to drip through a narrow bore hole or tube. The time it takes a defined amount of the mixture to drip through the narrow bore hole or tube is then correlated with SCC. Correlation may be carried out by any means, for example a standard curve of sec and drip times, or by means of a pre-programmed processor which is adapted to receive data relating the drip time and correlate the data with a SCC estimate. A macro-scale dripping device, and kit of parts, suitable for performing the method of the invention are also provided.

However, similar to WO 01/35728 A1, WO 2010/079469 A1 neither discloses nor suggests the implementation of the test in a microfluidic device and is silent about the challenges and benefits such implementation provides.

### SUMMARY OF THE INVENTION

The present inventor has observed that traditional quantification of somatic cells of biological sample tends to involve a lot of manual handling, which lead to imprecise results. Alternatively, traditional quantification of somatic cells of biological samples requires expensive, sophisticated equipment that works well in central laboratories or is integrated in automated milking systems (e.g. WO 01/35728 A1) but is unsuited for cow-side testing (see e.g. Figure 12 of WO 01/35728 A1).

The present inventor has discovered that mastitis testing based on the presence of somatic cells in the raw milk advantageously can be performed in a microfluidic device comprising a micro conduit system which contains the chemical agents that form DNA gels in macroscale. It is well-known that the handling of highly viscous liquids in microfluidic devices is challenging and that the formation of gel lumps in a microfluidic device may cause clogging. While one might expect that the formation of gel in a micro conduit system makes the handling of the system unpredictable and prone to clogging, the inventor has found that the viscosity change associated with the DNA gelling in the micro conduit system may in fact be used for determining the level of DNA-containing cells in the liquid sample provided to the micro conduit system.

The inventor has furthermore discovered that variation in the basic viscosity of milk from single cow or between cows, e.g. due to seasonal variations or change in diet, causes imprecision when attempting to convert the prior art SCC method to micro device format.

The inventor has furthermore found that this problem can be solved by measuring, in the microfluidic device, both liquid sample that has been exposed to DNA gelling agent and liquid sample that has not been exposed to DNA gelling agent. The inventor has also found that the problem alternatively can be solved by measuring one or more parameter(s) that already at least partially compensates for the variation in the basic viscosity of the liquid sample to be tested.

Additionally, the inventor has found that the liquid handling during testing which is applied in the prior art can be simplified significantly by providing the DNA gelling system in dry form in the micro conduit system of the microfluidic device instead providing it as a separate liquid inflow that has to be mixed with the liquid sample.

Thus, an aspect of the invention pertains to a method of determining the level of DNA-containing, biological cells of a biological source, the method comprising the steps of:
a) providing a microfluidic device comprising:
   - a sample reception structure,
   - a micro conduit system comprising a DNA gelling system comprising a DNA gelling agent, wherein the DNA gelling system is present in dry form,
b) adding a liquid sample of the biological source to the sample reception structure,
c) contacting the DNA gelling system with a first portion of the sample, thereby generating DNA gel, and
d) measuring a parameter correlated with the viscosity of the first portion of the sample during and/or after contacting the first portion with the DNA gelling system,
e) optionally, measuring a parameter correlated with the viscosity of a portion of the sample which has not been in contact with the DNA gelling system, and
f) determining the level of DNA-containing, biological cells of the biological source using the measurements of step d) and optionally also e), and
   wherein
   - the method includes step e) and uses the measured parameter of step e) in the determination of step f) and/or
   - the measured parameter of step d) at least partially compensates for variation in the viscosity of the liquid sample.

The method of determining the level of DNA-containing, biological cells of a biological source may be perceived as a method of testing a biological source to determine the level of DNA-containing, biological cells contained by the biological source.

Another aspect of the invention pertains to a type A microfluidic device which is useful for the above-described method, that is, for determining the level of DNA-containing cells in a biological sample, the microfluidic device comprising:
- a sample reception structure,
- a micro conduit system comprising a first micro conduit section in fluid communication with the sample reception structure, said micro conduit section comprising a DNA gelling system comprising a DNA gelling agent
- means for measuring a parameter correlated with the viscosity of a portion of the sample which has not been in contact with the DNA gelling agent,
- means for measuring a parameter correlated with the viscosity of a portion of the sample which has been in contact with the DNA gelling system.

The present inventor has furthermore discovered a particularly useful system comprising a specific microfluidic device and a liquid metering component, which system advantageously can be used to implement the above-mentioned method. The system has the advantages that it is simple to produce, it is very robust and easy to use and can provide rapid information on the somatic cell number of milk from individual cows or even individual udders. It furthermore allows for visual read-out of the results and the read-out is available and stable for at least 30 minutes.

Thus, yet an aspect of the invention pertains to a system for determining a change of viscosity in a liquid sample, the system comprising:
- a type B microfluidic device including a micro conduit system, the type B microfluidic device comprising:
   - a sample reception structure,
   - a micro conduit system comprising:
      - an agent capable of providing a change of viscosity in a liquid sample containing a target entity,
      - a first micro conduit section, said first microfluidic conduit section containing the agent,
      - a second micro conduit section which is substantially free of the agent,
      - both the first and the second micro conduit sections are so arranged that that a liquid sample can enter them simultaneously,
   - means for inspecting the presence or non-presence of liquid in at least one position in at least one of the first and second micro conduit sections, and preferably at least one position in each of the first and second micro conduit sections, and
- a liquid metering component capable providing a pre-determined volume of a liquid sample.

A further aspect of the invention pertains to a method of determining the level of a target entity in a liquid sample, the method comprising the following steps:
i) providing a system or a type B microfluidic device as defined herein,
ii) applying a volume, and preferably a pre-determined volume, of liquid sample to the sample reception structure,
iii) providing the liquid sample access to flow into the first and second micro conduit sections at the same time, thereby allowing the two sections to compete for the liquid sample, and
iv) detecting the presence or non-presence of liquid in at least one position in at least one of the first and second micro conduit sections, and preferably at least one position in each of the first and second micro conduit sections,
v) determining the level of target entity in the liquid sample based on the detection(s) of step iv).

A broader aspect of the invention pertains to a method of determining the level of DNA-containing, biological cells of a biological source, the method comprising the steps of:
a) providing a microfluidic device comprising:
   - a sample reception structure,
   - a micro conduit system comprising a DNA gelling system comprising a DNA gelling agent,
b) adding a liquid sample of the biological source to the sample reception structure,
c) contacting the DNA gelling system with a first portion of the sample, thereby generating DNA gel, and
d) measuring a parameter correlated with the viscosity of the first portion of the sample during and/or after contacting the first portion with the DNA gelling system,
e) optionally, measuring a parameter correlated with the viscosity of a portion of the sample which has not been in contact with the DNA gelling system, and
f) determining the level of DNA-containing, biological cells of the biological source using the measurements of step d) and optionally also e).

### SUMMARY OF THE FIGURES

Fig. 1 contains a schematic illustration of a type B microfluidic device (1) containing a sample reception structure (2) in direct fluid communication with a first micro conduit section (3) and a second micro conduit section (4). The first micro conduit section (3) contains a DNA gelling system (5) dried on the walls of the part of the first micro conduit section that is closest to the sample reception structure.
Fig. 2 is a schematic illustration of another type B microfluidic device (1) containing a sample reception structure (2) in direct fluid communication with a channel (6) that feeds both the first micro conduit section (3) and a second micro conduit section (4). The lid covering the micro conduit system is not shown.
Fig. 3 is a schematic illustration of another type B microfluidic device (1) containing a sample reception structure (2) in direct fluid communication with a first micro conduit section (3) and a second micro conduit section (4). Again, the first micro conduit section (3) contains a DNA gelling system (5) in dry form. The micro conduit system is covered by a non-transparent lid (7), which lid contains some optical windows (8a, 8b, 8c, 8r₁ and 8r₂).
Fig. 4 is a schematic illustration of yet another type B microfluidic device (1) which is similar to the device of Fig. 3, but which has a transparent lid (7) and a scale (9) for estimating the cell number of the liquid sample.
Fig. 5 shows a top view of a type B microfluidic device (1) containing a sample reception structure (2) and a micro conduit system comprising a first micro conduit section (3) used as a measurement-channel and a second micro conduit section (4) used as a reference-channel.
Fig. 6 shows a top view of the type B microfluidic device (1) of Fig. 5 marking approximately where the liquid front of the liquid sample would stop as a function of the cell number of the sample (cells/mL).
Fig. 7 shows a top view of another type B microfluidic device (1) wherein DNA-gel-capturing micro pillars have been positioned along the entire length of the first micro conduit section.
Fig. 8 shows a top view of yet another type B microfluidic device (1) which resembles that of Fig. 7 but which furthermore contains a channel constriction (11) in the second micro conduit section.
Fig. 9 shows a top view of a type B microfluidic device (1) comprising a backflow-reducing structure (12).
Fig. 10 is a microscopy picture of a portion of a first micro conduit section containing micro pillars. The first micro conduit section contained a nearly cell-free milk sample which has contacted a DNA gelling system (SDS).
Fig. 11 is a microscopy picture of a portion of a first micro conduit section containing micro pillars. The first micro conduit section contains a milk sample which has a somatic cell count of approx. 300,000 cells/mL, which milk sample has contacted a DNA gelling system (SDS).

### DETAILED DESCRIPTION OF THE INVENTION

An aspect of the invention pertains to a method of determining the level of DNA-containing, biological cells of a biological source, the method comprising the steps of:
a) providing a microfluidic device comprising:
   - a sample reception structure,
   - a micro conduit system comprising a DNA gelling system comprising a DNA gelling agent, wherein the DNA gelling system is present in dry form,
b) adding a liquid sample of the biological source to the sample reception structure,
c) contacting the DNA gelling system with a first portion of the sample, thereby generating DNA gel, and
d) measuring a parameter correlated with the viscosity of the first portion of the sample during and/or after contacting the first portion with the DNA gelling system,
e) optionally, measuring a parameter correlated with the viscosity of a portion of the sample which has not been in contact with the DNA gelling system, and
f) determining the level of DNA-containing, biological cells of the biological source using the measurements of step d) and optionally also e), and
   wherein
   - the method includes step e) and uses the measured parameter of step e) in the determination of step f) and/or
   - the measured parameter of step d) at least partially compensates for variation in the viscosity of the liquid sample.

Step c) is performed in the microfluidic device.

Steps d) and e) are preferably also performed in or on the microfluidic device.

Step f) may be performed in the microfluidic device or outside the microfluidic device. For example, the person performing the method may make the measurement of step d) and optionally also of step e) by visual inspection of the microfluidic device and determine the position of the liquid front and/or the presence or non-presence of liquid at pre-selected positions of first and/or second micro conduit sections.

The present inventor has found that by using the above method and/or the novel microfluidic devices presented herein it is possible to provide a simple, precise and robust test system which does not depend on complex valve operation schemes for automated timing that is used in the prior art.

It is furthermore believed that the microscale dimensions used in the micro conduit system of the present microfluidic devices provide short diffusion-distances and high surface-to-volume - ratios which provide for the very short reaction-time scales that allow for rapid testing.

In the context of the present invention the term "DNA-containing, biological cells" pertains to biological cells, e.g. eukaryote cells or prokaryotic cells, that contain packaged, double stranded DNA.

In the case of eukaryote cells, such as somatic cells, the DNA is preferably present in packaged form, that is, in histone-stabilised form and e.g. forming supercoils. The DNA may for example be in chromatin conformation.

In the case of prokaryote cells the DNA is preferably present in packaged form, and e.g. in coiled form facilitated by bacterial DNA binding proteins.

The DNA-containing, biological cells may for example be somatic cells found in raw milk samples or white blood cells found in blood samples.

In some preferred embodiments of the invention, the method of determining the level of DNA-containing, biological cells of a biological source may for example be a method of determining the level of somatic cells in raw milk. In this case the liquid sample may comprise, or even essentially consists of, raw milk. The raw milk is preferably freshly milked, unpasteurised and/or non-homogenised.

The raw milk may be from mammals such as cows, buffalos, goats, sheep, camels or llamas. In some preferred embodiments the raw milk is bovine milk.

In the context of the present invention, the term "biological source" pertains to the source from which the liquid sample has been derived. The liquid sample comprises at least some of the double-stranded DNA of the biological source and it may e.g. be the biological source as such. However, the liquid sample preferably contains no or only insignificant amounts of gelled DNA.

In some preferred embodiments of the invention, the biological source comprising DNA-containing cells is a milk sample, preferably a raw milk sample, such as a sample of untreated raw milk.

In other preferred embodiments of the invention, the biological source comprising DNA-containing cells is a blood sample, a urine sample, a saliva sample, a food sample, an environmental sample, or a sample from an industrial fermentation process.

A sample from an industrial fermentation process may for example be a sample stemming from a production of food or medicine which involves fermentation.

For example, the biological source comprising DNA-containing cells may be a blood sample, a urine sample, or a saliva sample.

Step a) involves providing a microfluidic device comprising:
- a sample reception structure, and
- a micro conduit system comprising a DNA gelling system comprising a DNA gelling agent.

In the context of the present invention, the term "microfluidic device", sometimes also referred to as a micro-TAS (micro-total analysis) device, a lab-on-a-chip device, or a biochip device, pertains to a device containing at least one micro conduit system adapted for handling of aqueous liquids. Such microfluidic devices can be produced in a multitude of different materials such as e.g. silicon, glass, ceramics and/or organic polymers, such as thermoplastic polymers.

The terms "type A microfluidic device" and "type B microfluidic device" refers to two specific aspects or embodiments of a microfluidic device. The type A microfluidic device is specific in the sense that it contains a DNA gelling system whereas the type B microfluidic device is specific in the sense that it contains a first micro conduit section and a second micro conduit section which compete for the liquid sample applied to the sample reception structure. The first micro conduit section of the type B microfluidic device furthermore comprises an agent capable of providing a change of viscosity in a liquid sample containing a target entity. This agent may be a DNA gelling system, in which case the type B microfluidic device is a preferred embodiment of type A microfluidic device. Features and embodiments which are described in the context of a microfluidic device as such apply to both the type A microfluidic device and the type B microfluidic device. A feature which is described in the context of the type A microfluidic device may also be used together with the type B microfluidic device unless it is clear that the feature described in the context of the type A microfluidic device cannot be used for the type B microfluidic device. A feature which is described in the context of the type B microfluidic device may be also be used together with the type A microfluidic device unless it is clear that the feature described in the context of the type B microfluidic device cannot be used for the type A microfluidic device.

The microfluidic device may be prepared in many different ways using e.g. conventional microtechniques such as micro-fabrication, micro-milling, injection molding, hot embossing, and laser machining. The device may be implemented as a highly complex, multilayered structure or as a simple structure comprising few layers. Relatively simple devices comprising as few device parts as possible are presently preferred. The device parts are the parts of which the device is assembled, and in some preferred embodiments of the invention, the device parts comprise a lid and a base portion. The base portion typically comprises a surface into which the micro conduit system has been imprinted or otherwise created, and onto which surface the lid has to be attached to complete the micro conduit system. In this case, the walls of the micro conduit system are partly or completely formed by the base portion and the lid.

In some preferred embodiments of the invention the microfluidic device is formed by two layers, i.e. a base portion in which a micro conduit system, which is to open to one side, has been formed, and a lid.

In some preferred embodiments of the invention, the microfluidic device is formed by three layers, such as a base portion in which a micro conduit system, which is open to two sides, has been formed, and an upper lid and a lower lid attached to the top and bottom of the base portion. In this way, at least a part of the base portion is sandwiched between the upper and lower lids.

The microfluidic device may have a number of different shapes and dimensions. For example, the microfluidic device may have dimensions comparable to a credit card or a microscopic slide. The microfluidic device may e.g. have a thickness in the range of 0.3-20 mm, a width in the range of 1-10 cm and a length in the range of 3-20 cm.

Alternatively, the microfluidic device may e.g. have a thickness in the range of 1-10 mm, a width in the range of 1-5 cm and a length in the range of 5-15 cm.

The microfluidic device may e.g. have a thickness in the range of 1-5 mm, a width in the range of 1-3 cm and a length in the range of 5-10 cm.

Alternatively, the microfluidic device may e.g. have a thickness in the range of 1-5 mm, a width in the range of 3-5 cm and a length in the range of 4-8 cm.

In some embodiments of the invention, the microfluidic device is a disc having a thickness in the range of 1-20 mm and having a radius in the range of 3-10 cm. For example, the microfluidic device may be a disc having a thickness in the range of 1-5 mm and having a radius in the range of 4-8 cm.

In the context of the present invention, the term "sample reception structure" pertains to the part of the microfluidic device where the liquid sample is supposed to be loaded. The sample reception may be designed as an open chamber capable of receiving a volume of liquid sample which is at least that required to perform the method.

Alternatively, the sample reception structure may be a narrow opening designed precisely to receive a pipette-like component and guide the liquid sample that the pipette-like component holds or provides into the micro conduit system.

The sample reception structure is typically in fluid communication with at least one section of the micro conduit system, e.g. by one or more micro channels of the micro conduit system which are capable of receiving liquid sample provided to the sample reception structure.

In the context of the present invention, the term "fluid communication" used in connection with micro conduit components or other structures related to the microfluidic device encompasses the alternatives "direct fluid communication" or "indirect fluid communication". The term "direct fluid communication" means that a gas molecule present in the holding volume of a first structure or conduit is capable of diffusing into the holding volume of a second structure or conduit without moving through solid material. In the case of "direct fluid communication", the first structure or conduit may adjoin the second structure or conduit and the fluid communication takes place through an opening between the two structures or conduits. Alternatively, the first and second structures or conduits may be connected by one or more additional micro conduit components such as e.g. a micro channel having both an opening to the first structure or conduit and an opening to the second structure or conduit.

The term "indirect fluid communication" means that a gas molecule present in the holding volume of a first structure or conduit cannot diffuse into the holding volume of a second structure or conduit without moving through solid material, unless one or more micro conduit components are operated to allow for the diffusion. In this case the one or more micro conduit components may be one or more micro valves or flow barriers that have to be broken or opened.

In the context of the present invention, the term "micro conduit system" relates to one or more micro conduit components comprised by the microfluidic device. If the micro conduit system contains more than one micro conduit component these micro conduit components are preferably in direct or indirect fluid communication. Preferably, a micro conduit system comprises at least two micro conduit components.

Typical micro conduit components are micro channels, micro chambers, micro filters, micro pillars, micro pillar arrays, micro cuvettes, micro mixers, micro pumps and/or micro valves. A micro conduit component typically has a smallest cross sectional dimension in the range of 10 - 5^{∗}10³ micron, preferably in the range 20 -3^{∗}10³ micron, and even more preferred in the range 50 - 2^{∗}10³ micron.

Even smaller components are possible, thus a micro conduit component may e.g. have a smallest cross sectional dimension in the range of 10 - 1^{∗}10³ micron, preferably in the range 20 - 0.8^{∗}10³ micron, and even more preferred in the range 50 - 0.5^{∗}10³ micron.

The microfluidic device may contain a single micro conduit system or a plurality of micro conduit systems.

In some preferred embodiments of the invention, the microfluidic device, e.g. the type A microfluidic device or the type B microfluidic device, comprises 4 micro conduit systems connected to each their sample reception structure, each of the 4 micro conduit system capable of the determining the level of somatic cells in a raw milk sample. Such a microfluidic device is particularly useful for cow-side testing as it allows for testing each quarter of the cow.

In the context of the present invention, the phrase "Y and/or X" means "Y" or "X" or "Y and X". Along the same line of logic, the phrase "n₁, n₂, ..., nᵢ₋₁, and/or nᵢ" means " n₁" or " n₂" or ... or "nᵢ₋₁" or "nᵢ" or any combination of the components : n₁, n₂,...nᵢ₋₁, and nᵢ.

In the context of the present invention, the term "conduit section" relates to a part of the micro conduit system, such as e.g. a specific micro conduit component or a number of connected micro conduit components.

The micro conduit system may comprise at least one micro channel. The micro conduit system may comprise at least one micro chamber. The micro conduit system may comprise at least one filter. The micro conduit system may comprise at least one electrode and preferably a pair of electrodes.

In some embodiments of the invention, the micro conduit system furthermore comprises at least one hydrophobic section.

In the context of the present invention, the term "hydrophobic section" relates to a section of the micro conduit system, in which the surface is hydrophobic.

A surface is hydrophobic if the contact angle between a drop of demineralised water and the surface is more than 90 degrees. The contact angle is determined as the angle, measured inside the drop of demineralised water, between the surface-water interface and the water-air interface.

The microfluidic device used in the method of determining the level of DNA-containing, biological cells of a biological source may for example be one of the microfluidic devices described herein, that is, the type A microfluidic device or the type B microfluidic device.

Alternatively, the microfluidic device may be a microfluidic device according to WO2008083687 (A1) - replacing the coagulation reagent and/or the agglutination reagent with the present DNA gelling system, e.g. an anionic surfactant such as e.g. SDS or a similar surfactant.

In the context of the present invention, the term "DNA gelling system" pertains to a chemical or biological system comprising at least a DNA gelling agent which is capable of generating a DNA gel when contacting DNA in chromatin conformation. The DNA gelling agent may furthermore be able to lyse DNA-containing cells, such as e.g. somatic cells, yeast cells or bacteria. Preferably, the DNA gelling agent is capable of lysing eukaryote and/or prokaryote cells.

The DNA gelling system may e.g. be present in the microfluidic device in liquid dissolved form or in dried form. A dried DNA gelling system is presently preferred.

In the present context, the terms "dry" and "dried" are used interchangeably.

A dry or dried DNA gelling system preferably contains at most 10% (w/w) water, preferably at most 6% (w/w) water, and even more preferably at most 4% (w/w) water.

In some preferred embodiments of the invention, the DNA gelling agent comprises, or even essentially consists of, an anionic surfactant and/or a cationic surfactant.

In other preferred embodiments of the invention, the DNA gelling agent comprises, or even essentially consists of, an anionic surfactant.

The DNA gelling agent may e.g. comprise, or even essentially consist of, an alkyl sulfate salt and/or an aryl sulfate salt. A useful example of an alkyl sulfate salt is a dodecyl sulfate salt, such as e.g. SDS.

Alternatively, the DNA gelling agent may e.g. comprise, or even essentially consist of, an alkyl sulfonate salt and/or an aryl sulfonate salt.

The DNA gelling system may furthermore comprise a cell lysing agent which is different from the DNA gelling agent. This is particularly useful when the DNA gelling agent is not capable of lysing the DNA containing cells.

In some embodiments of the invention, the DNA gelling agent comprises, or even essentially consists of, an alkalizing agent, such as e.g. a hydroxide salt. The DNA gelling system may for example comprise, or even essentially consist of, an alkalizing agent.

Examples of such an alkalizing agent could e.g. be sodium hydroxide or potassium hydroxide.

In some preferred embodiments of the invention, the DNA gelling system comprises both an anionic surfactant and an alkalizing agent.

The weight ratio between the total amount of anionic surfactant and total amount of alkalizing agent is preferably in the range of 1:10 - 10:1. For example, the weight ratio between the total amount of anionic surfactant and total amount of alkalizing agent may be in the range of 1:2 - 6:1. Alternatively, the weight ratio between the total amount of anionic surfactant and total amount of alkalizing agent may be in the range of 1:1 - 3:1, such as e.g. approx. 2:1.

The cell lysing agent may for example be a surfactant.

The DNA gelling system is preferably present in the micro conduit system in an amount sufficient to form DNA gel in the first portion of the liquid sample in at most 5 minutes. For example, the DNA gelling system may be present in the micro conduit system in an amount sufficient to form DNA gel in the first portion of the liquid sample in at most 3 minutes. Alternatively, the DNA gelling system may be present in the micro conduit system in an amount sufficient to form DNA gel in the first portion of the liquid sample in at most 1 minute.

Preferably, the DNA gelling system is present in the micro conduit system in an amount sufficient to form DNA gel in the first portion of the liquid sample in at most 0.5 minutes.

The inventor has found that the required dosage of the DNA gelling system depends on the design of the micro conduit system. The required dosage of the DNA gelling system may also be dependent of the range and/or resolution of the intended sample to be characterized. E.g. one system may be intended for use in connection with high cell-concentrations, where a relatively lower dosage of DNA gelling system may provide the optimal results, whereas one may require a higher dosage of DNA gelling system in case low cell-concentrations are to be characterized. The DNA gelling system may for example be present in an amount sufficient to provide a concentration of the DNA gelling system in the first portion of the liquid sample in the range of 0.05-20% (w/w), such as e.g. in the range of 0.1-15% (w/w), or in the range of 1-10% (w/w), such as in the range of 5-10% (w/w).

In some preferred embodiments of the invention, the micro conduit system contains in the range of 0.05-50 mg DNA gelling system, preferably anionic surfactant. For example, the micro conduit system may contain in the range of 0.1-30 mg DNA gelling system. Alternatively, the micro conduit system may contain in the range of 0.5-10 mg DNA gelling system. The present inventor has found that an advantage of the present methods and microfluidic device is that they use less DNA gelling reagents than conventional testing for somatic cells and that the reagents furthermore are contained inside the microfluidic device and not poured in the sewer after use. Several DNA gelling agents, e.g. some anionic surfactants such as SDS, have been shown to be toxic to fish and wild-life and the present invention reduces the pollution provided by such agents.

The micro conduit system may furthermore contain other chemical components in addition to the DNA gelling system.

The means for measuring a parameter correlated with the viscosity of a portion of the sample which has been in contact with the DNA gelling system is preferably located down-stream of the place in the micro conduit system where the DNA gelling system has been deposited or at the place in the micro conduit system where the DNA gelling system has been deposited.

The micro conduit system may for example comprise flow-aiding surfactants which aid the flow of the liquid sample in the micro conduit system. Such flow-aiding surfactants are preferably not DNA gelling agents and may for example be chosen from non-ionic surfactants. Non-limiting examples of useful non-ionic surfactants are polysorbates such as e.g. Tween-20, polyethylene oxide-derived surfactants such as e.g. Triton-X100), polyethylene glycol(PEG)-type surfactants, or alkyl phenoxypolyethoxyethanols such as e.g. nonvlphenoxypolyethoxyethanol, or a combination thereof.

Alternatively, or additionally, the micro conduit system or one or more sections of the micro conduit system may additionally have been subjected to hydrophilic surface treatments, such as e.g. surface treatment by acids or by bases, plasma treatment, treatment involving molecular vapour deposition (MVD), or a combination thereof.

The micro conduit system may furthermore comprise one or more dyes and/or pigments to increase the visibility of one or more liquids in the microfluidic device. Dyes and/or pigments are particularly useful when the measurements are based on visual inspection of the position of liquid in a part of the micro conduit system or the presence or non-presence of liquid at a specific position in the micro conduit system. This is particularly useful in the context of the type B microfluidic device.

Step b) involves adding a liquid sample of the biological source to the sample reception structure.

In the context of the present invention, the term "liquid sample of the biological source" pertains to a liquid sample which may be the biological sample as such or at least contains a substantial amount of the chromatin or somatic cells of the biological source.

The biological source may have been subjected to pre-treatment in order to prepare the liquid sample. For example, the liquid sample may contain biological sample diluted in a liquid, such as e.g. an aqueous pH buffer solution. Alternatively, the liquid sample may contain a subset of the components present in the biological source. For example, interfering entities may have been removed while converting the biological source into the liquid sample.

In some preferred embodiments of the invention, the liquid sample is raw milk or diluted raw milk.

In other preferred embodiments of the invention, the liquid sample is whole blood or diluted whole blood.

In yet other embodiments of the invention, the liquid sample is saliva or diluted saliva.

In further preferred embodiments of the invention, the liquid sample is urine or diluted urine.

The liquid sample may alternatively comprise or even consist of a lysate of the biological source, e.g. a sample wherein the biological cells have been lysed, e.g. chemically or physically, but wherein the DNA is still in its packaged, and e.g. super-coiled, structure.

Step c) involves contacting the DNA gelling system with a first portion of the sample, thereby generating DNA gel.

In the context of the present invention, the term "contacting the DNA gelling system" means that a liquid, e.g. the first portion of the liquid sample, is interacting with the DNA gelling system. If the DNA gelling system contains components which are soluble in the liquid, at least some of these soluble components will dissolve and mix into the liquid. This is for example the case when the DNA gelling system comprises, or even consists of, an anionic surfactant and/or an alkalizing agent.

In the context of the present invention, the term "first portion of the sample" pertains to a sub-sample of the liquid sample which sub-sample initially may have substantially the same chemical composition as the liquid sample, or which initially at least contains some of the DNA on packaged, and e.g. super-coiled, form and/or some of the DNA-containing cells of the liquid sample.

In the context of the present invention, the term "DNA gel" pertains to the gel-like liquid which is formed when packaged or super-coiled DNA is exposed to an anionic surfactant, such as SDS, or a similar DNA gelling agent. Without being bound by theory, it is believed that the gel is formed by at least partially or even completely unpackaged DNA, which provides an increase of the viscosity relative to packed DNA, and therefore gives rise to a gel-like consistency of the liquid wherein it is contained. SDS is believed to destabilise the histone complexes (or similar DNA binding proteins) that stabilise DNA in its packaged structure. This protein destabilization is believed to cause the at least partial unpackaging of the DNA.

Step d) involves measuring a parameter correlated with the viscosity of the first portion of the sample during and/or after contacting the first portion with the DNA gelling system,

In the context of the present invention, the term "measuring" relates to recording or reading the value of the parameter. The measurement may be performed manually by visual inspection or automatically. The automatic measurement may e.g. be performed inherently by the microfluidic device. Alternatively, an automatic measurement may be made by an apparatus which is capable of performing the measurement(s) of step d) and/or e), e.g. via an optical or electrical interface.

In the context of the present invention, the term "parameter correlated with the viscosity" pertains to a parameter which can be the viscosity as such or a parameter which correlates with the viscosity of the liquid in question. The parameter has to change when the viscosity of the liquid changes. For example, the parameter may increase when the viscosity increases and decrease when the viscosity decreases. Alternatively, the parameter may increase when the viscosity decreases and decrease when the viscosity increases.

The parameter correlated with the viscosity of the first portion of the sample may for example be one of the following:
i) the flow rate of the first portion of the sample during and/or after contact with the DNA gelling system,
ii) the steady-state position of a liquid in the microfluidic device, which liquid is or has been in liquid contact with the first portion of the sample during and/or after contact with the DNA gelling system,
iii) presence or non-presence of liquid at a specific location in the microfluidic device, which liquid is or has been in liquid contact with the first portion of the sample during and/or after contact with the DNA gelling system, and
iv) an optical property of the first portion of the liquid sample.

Examples of optical properties are e.g. the turbidity of the first portion of the liquid sample, absorption of the first portion at at least one wavelength, the refractive index of the first portion, or the ability of the first portion to reflect or refract electromagnetic radiation at a given wavelength.

The inventor has found that the basic viscosity of the liquid sample surprisingly is a significant source of imprecision when the level of DNA-containing cells are measured in microfluidic devices, particularly if the liquid handling in the microfluidic device is based on capillary forces.

In some preferred embodiments of the invention, the measured parameter of step d) furthermore at least partially compensates for variation of the viscosity of the liquid sample, i.e. variation that is not related to DNA gelling or the content of DNA-containing cells.

In the context of the present invention, a parameter which "at least partially compensates for variation of the viscosity of the liquid sample" is a parameter wherein changes of the basic viscosity of liquid samples (without DNA gelling) has less weight than change cause by DNA gelling. Preferably the parameter measured in step d) primarily correlates to changes in the viscosity due to DNA gelling and is at least partially insensitive to the viscosity of the liquid sample as such. Even more preferred, the parameter measured in step d) is substantially insensitive to the viscosity of the liquid sample as such.

The type B devices described herein allows for step d) measurements that inherently compensate for at least some of the variation of the viscosity of the liquid sample. Thus, in some preferred embodiment of the method of determining the level of DNA-containing, biological cells of a biological source, the method comprising the steps of:
a) providing a microfluidic device comprising:
   - a sample reception structure,
   - a micro conduit system comprising a DNA gelling system comprising a DNA gelling agent, wherein the DNA gelling system is present in dry form,
b) adding a liquid sample of the biological source to the sample reception structure,
c) contacting the DNA gelling system with a first portion of the sample, thereby generating DNA gel, and
d) measuring a parameter correlated with the viscosity of the first portion of the sample during and/or after contacting the first portion with the DNA gelling system,
e) optionally, measuring a parameter correlated with the viscosity of a portion of the sample which has not been in contact with the DNA gelling system, and
f) determining the level of DNA-containing, biological cells of the biological source using the measurements of steps d) and optionally also e), and
   wherein
   - the method includes step e) and uses the measured parameter of step e) in the determination of step f) and/or
   - the microfluidic device is a type B microfluidic device.

In some preferred embodiments of the invention, the method comprises step e). The present inventor has found that the reference measurement provided by step e) increases the accuracy of the method.

Thus, in some preferred embodiments of the invention, the method comprises a step of measuring a parameter correlated with the viscosity of a portion of the sample which has not been subjected to DNA gelling.

For example, the portion of the sample which has not been subjected to DNA gelling may be a portion which has not been in contact with the DNA gelling system or with the DNA gelling agent of the DNA gelling system.

The portion of the sample which has not been subjected to DNA gelling may for example be the first portion before it is contacted with the DNA gelling agent or with the DNA gelling agent of the DNA gelling system.

Alternatively, the measurement of a parameter correlated with the viscosity of a portion of the sample which has not been in contact with the DNA gelling system may involve measuring the parameter on a portion of the sample that is different from the first portion.

In some preferred embodiments of the invention, the parameter correlated with the viscosity measured in step e) is a parameter of the same type as that measured in step d).

The method may optionally include step e) of measuring a parameter correlated with the viscosity of a portion of the sample which has not been in contact with the DNA gelling system.

In some preferred embodiments of the invention, the method includes a step e) of measuring a parameter correlated with the viscosity of a portion of the sample which has not been in contact with the DNA gelling system.

Step f) involves determining the level of DNA-containing, biological cells of the biological source using the measurements of steps d) and optionally also e).

In the context of the present invention, the term "determining the level of DNA-containing biological cells of the biological source" pertains to determining at least whether the level of biological cells of the source is above a certain threshold level and optionally also whether it is at the threshold level or whether it is below the threshold level. Alternatively, the determination may involve the determination of the number of biological cells of the biological source per volume unit or weight unit of the biological source.

Thus, in some preferred embodiments of the invention, the determination of the level of DNA-containing biological cells of the biological source involves at least a binary determination of whether the level of DNA-containing biological cells is:
- above, or
- below
   a predefined threshold level.

The determination of the level of DNA-containing biological cells of the biological source may e.g. involve determination of whether the level of DNA-containing biological cells is within at least 2 different ranges, such as for example at least three different ranges.

Alternatively, the determination of the level of DNA-containing biological cells of the biological source may involve a determination of the exact number of cells per volume or weight unit of the biological source.

The determination preferably involves the use of a calibration set which converts the measurement(s) performed in step d) and optionally also e) to the number of biological cells or biological cell equivalents of the liquid sample. If the liquid sample is identical to the biological source, the number of biological cells found in the liquid sample is the same as in the biological source.

If the liquid sample has been derived from the biological source by pre-treatment such as dilution and e.g. other manipulations, the number of biological cells found in the liquid sample should be corrected appropriately to arrive at the number of cells in the biological source. For example, if the pre-treatment resulting in the liquid sample involved a factor 2 dilution of the biological source (e.g. diluting 1 mL raw milk to obtain a 2 mL liquid sample), the concentration of cells found in the liquid sample should be multiplied by a factor of 2 to arrive at the number of cells of the biological source.

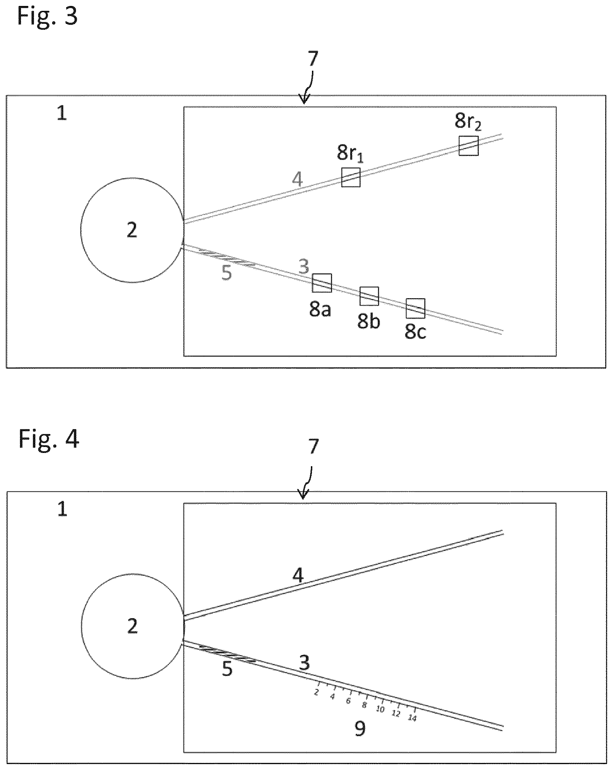

In some preferred embodiments of the invention, the calibration set is shown directly on the microfluidic device so that the user can determine the level of DNA-containing biological cells directly by looking at the microfluidic device when the test has been performed. See for example the type B microfluidic devices of Fig. 3 or Fig. 4 and the description of the depicted embodiments.

Alternatively, the calibration set may be supplied separately but is available to the person performing the method and using the microfluidic device.

In some preferred embodiments of the invention, the calibration set is stored in the memory of a microprocessor, e.g. internal memory of the microprocessor or memory which is accessible to the microprocessor. The microprocessor obtains the parameter(s) measured in step d) and optionally also in step e), e.g. by performing the measurements itself, and determines the level of DNA-containing, biological cells of the biological source by using an algorithm that combines the calibration set with the measurement(s). Such algorithms are well-known to the person skilled in the art, e.g. in the form of calibration curves.

The microprocessor may form part of the microfluidic device or it may form part of a reader apparatus.

In some preferred embodiments of the invention, the determination of the level of DNA-containing biological cells of the biological source and the measurement is performed by visual inspection of the microfluidic device.

Alternatively, if the measurement(s) are performed electrically or optically the determination of the level of DNA-containing biological cells of the biological source may be performed by a microprocessor based on the measurement(s) of step d) and optionally also step e).

The microprocessor may e.g. form part of the microfluidic device or it may form part of a reader device capable of performing the measurements or at least reading the parameters of step d) and optionally also step e).

The method may be performed at a broad range of temperatures, typically in the range of 0-90 degrees C. However, in some preferred embodiments of the invention the temperature of the liquid sample is adjusted to a specific target temperature prior to step c), which target temperature is in the range of 1-60 degrees C, preferably in the range of 2-40 degrees C, and even more preferably in the range of 10-30 degrees C.

The method may for example involve that the temperature of both the microfluidic device and the liquid sample is adjusted to a specific target temperature prior to step c), which target temperature is in the range of 1-60 degrees C, preferably in the range of 2-40 degrees C, and even more preferably in the range of 10-30 degrees C. The specific target temperature is preferably maintained during the performance of the method.

Preferably, the temperature of the microfluidic device and the liquid sample does not deviate from the specific target temperature more than ±5 degrees C, and even more preferably no more than ±2 degrees C, such as e.g. no more than ±1 degrees C.

The method of determining the level of DNA-containing, biological cells of a biological source may e.g. be performed using the type B microfluidic device as microfluidic device.

Alternatively, the method of determining the level of DNA-containing, biological cells of a biological source may involve the use of the entire system for determining a change of viscosity in a liquid sample described herein, using a DNA gelling system as the agent capable of providing a change of viscosity in a liquid sample containing a target entity.

Another aspect of the invention pertains to a type A microfluidic device which is useful for the above-described method, that is, for determining the level of DNA-containing cells in a biological sample, the microfluidic device comprising:
- a sample reception structure,
- a micro conduit system comprising:
   - a first micro conduit section in fluid communication with the sample reception structure, said micro conduit section comprising a DNA gelling system comprising a DNA gelling agent
- means for measuring a parameter correlated with the viscosity of a portion of the sample which has not been in contact with the DNA gelling agent,
- means for measuring a parameter correlated with the viscosity of a portion of the sample which has been in contact with the DNA gelling system.

Examples of useful means for measuring a parameter correlated with the viscosity are:
- A micro flow meter,
- A measuring conduit section suitable for measuring the volume of liquid introduced into that section,
- One or more windows allowing for optical measurement,
- One or more windows allowing for the determining the position of a liquid in the micro conduit system at different time points,
- One or more windows allowing for the presence or non-presence of liquid at one or more positions of the micro conduit system, and
- One or more sensors allowing for the presence or non-presence of liquid at one or more positions of the micro conduit system.

It should be noted that the means for measuring a parameter correlated with the viscosity need not be a means for measuring the viscosity as such but can also be a means for measuring a parameter which correlates with the viscosity of the liquid in question. The parameter has to change when the viscosity of the liquid changes. For example, the parameter may increase when the viscosity increases and decrease when the viscosity decreases. Alternatively, the parameter may increase when the viscosity decreases and decrease when the viscosity increases.

A number of different liquid drives may be used for moving the liquid sample, and optionally also other liquids in the micro conduit system of the microfluidic device after the liquid sample has been introduced.

The liquid handling may for example involve one or more liquid drives selected from the group consisting of capillary forces, positive pressure, negative pressure, a liquid drive built into the microfluidic device, an external liquid drive.

In some preferred embodiments of the invention, the liquid drive comprises, or even essentially consists of, capillary forces. The advantage of capillary forces is that no external or internal pumping devices are required which reduces the complexity of the production of the microfluidic device.

In some preferred embodiments of the invention, the type microfluidic device is a type B microfluidic device.

In some preferred embodiments of the invention, the microfluidic device is disposable and intended for one time use. The microfluidic device may, however, contain multiple micro conduit systems, each connected to a separate sample reception structure which is independent from the other micro conduit systems, and which microfluidic device therefore is capable of performing multiple tests, i.e. one per micro conduit system.

For example, the microfluidic device may contain four micro conduit systems, each connected to a separate sample reception structure which is independent from the other micro conduit systems, and which microfluidic device therefore is capable of performing four tests, i.e. one per micro conduit system. Such as device is e.g. advantageous for mastitis testing and allows for separate testing of milk from each of the four teats of a cow.

Such a four test microfluidic device may furthermore comprise one or more indications and/or have a design which tells the user in which micro conduit system the milk from a specific teat should be tested. It should preferably be clear to the user:
- in which of the four sample reception structures the milk from the left front teat should be introduced,
- in which of the four sample reception structures the milk from the right front teat should be introduced,
- in which of the four sample reception structures the milk from the left rear teat should be introduced, and
- in which of the four sample reception structures the milk from the right rear teat should be introduced.

Another an aspect of the invention pertains to a system for determining a change of viscosity in a liquid sample, the system comprising:
- a type B microfluidic device including a micro conduit system, the microfluidic device comprising:
   - a sample reception structure,
   - a micro conduit system comprising:
      - an agent capable of providing a change of viscosity in a liquid sample containing a target entity,
      - a first micro conduit section, said first microfluidic conduit section containing the agent,
      - a second micro conduit section which is substantially free of the agent,
      - both the first and second micro conduit sections are so arranged that that a liquid sample can enter them simultaneously,
   - means for inspecting the presence or non-presence of liquid in at least one position in at least one of the first and second micro conduit sections, and preferably at least one position in each of the first and second micro conduit sections, and
- a liquid metering component capable of providing a pre-determined volume of a liquid sample.

The present inventor has discovered that both type A and type B microfluidic devices and the present methods are particularly useful for measuring the viscosity changes caused by the non-newtonian gels formed e.g. in low SCC milk and therefore has the potential of providing data for earlier detection of mastitis.

In some preferred embodiments of the invention, the liquid sample, such as e.g. raw milk, contains at most 2^{∗}10⁶ somatic cells per mL, preferably at most 1.5^{∗}10⁶ somatic cells per mL, and even more preferably at most 1.0^{∗}10⁶ somatic cells per mL.

In other preferred embodiments of the invention, the liquid sample, such as e.g. raw milk, contains at most 0.9^{∗}10⁶ somatic cells per mL, preferably at most 0.8^{∗}10⁶ somatic cells per mL, such as e.g. most 0.6^{∗}10⁶ somatic cells per mL.

The liquid sample, such as e.g. raw milk, may e.g. contain in the range of 0-1^{∗}10⁶ somatic cells per mL, preferably in the range of 0.01^{∗}10⁶- 0.9^{∗}10⁶ somatic cells per mL, and even more preferably in the range of 0.05^{∗}10⁶ - 0.8^{∗}10⁶ somatic cells per mL or in the range of 0.02^{∗}10⁶ - 0.6^{∗}10⁶ somatic cells per mL.

The terms "first micro conduit section" and "second micro conduit section" pertain to two micro conduit sections which are designed to compete for the liquid sample, when it has been introduced to the sample reception structure of the type B microfluidic device.

Preferably the volume of the liquid sample has been pre-determined so that neither the first micro conduit section nor the second micro conduit section can be completely filled during the competition.

The agent capable of providing a change of viscosity in a liquid sample containing a target entity may e.g. be a DNA gelling system.

The second micro conduit section is preferably substantially free of the agent capable of providing a change of viscosity in a liquid sample containing a target entity meaning that the agent may only be present in an insignificant amount which does not cause a change in viscosity.

For example, the second micro conduit section may e.g. be substantially free of the DNA gelling agent meaning that the DNA gelling agent may only be present in an insignificant amount which does not cause detectable DNA gelling.

While different liquid drives may be used in the type B microfluidic device, it is presently preferred to use capillary forces. Thus, in some preferred embodiments of the invention, the liquid drive of the type B microfluidic device comprises, or even essentially consists of, capillary forces.

The down-stream ends of the first and second micro conduit sections preferably each contain an opening or connection to the outside air in order to avoid trapping of air when the liquid sample enters the first and second micro conduit sections.

The first and second micro conduit sections may be designed so that the capillary flow of liquid in each section stops when the liquid sample has been distributed between the two sections during the competition. If the same cross-sectional dimensions are used throughout the first micro conduit section and the second micro conduit section, the flow tends to stop when the liquid sample of the sample reception structure has been distributed between the two micro conduit sections.

The downstream ends of the first and second micro conduit sections may in fact meet. However, at least when using the type B microfluidic device it is often preferred that the liquid sample never reaches any of the two joining down-stream section ends.

In the context of the present invention, the term "liquid metering component capable of providing a pre-determined volume of a liquid sample" pertains to:
- a component which, when used correctly, is only capable of providing a fixed pre-determined volume of liquid, or
- a component which controllably is capable of providing different pre-determined volumes of liquid including at least one pre-determined volume which is suitable for the microfluidic device.

In some preferred embodiments of the invention, the liquid metering component is a pipette or a pipette-type component capable of metering a pre-determined volume of a liquid sample to the sample reception structure. The pipette or a pipette-type component may e.g. be separate from the microfluidic device.

The liquid metering component may for example be an Exact Volume Transfer Pipettes (e.g. http://www.alphalabs.co.uk/product.aspx?p=1496).

The pipette or a pipette-type liquid metering component may for example be disposable. It may furthermore contain one or more auxiliary agents, such as a cell lysing agent, which preferably does not cause significant DNA gelling and/or a pH buffering agent.

In some embodiments of the invention, the following assay is used for determining whether a candidate agent at a given concentration is a DNA gelling agent:
A DNA stock solution of packaged DNA is prepared by preparing an aqueous suspension containing 400.000 bovine somatic cells/mL in 0.1 M phosphate buffer saline and 1% (w/w) Triton-X100. Triton-X100 1% lyses the cell membranes of the somatic cells but does not cause DNA gelling.

A candidate agent stock solution is prepared by dissolving the candidate agent in the buffer (0.1 M phosphate buffer saline incl. 1% (w/w) Triton-X100) in a concentration which is the double of the intended concentration for DNA gelling.

The following 4 samples are prepared:
A: 20 mL of the buffer (no candidate agent or DNA)
B: 10 mL DNA stock solution + 10 mL of the buffer
C: 10 mL of the buffer + 10 mL candidate agent stock solution
D: 10 mL DNA stock solution + 10 mL candidate agent stock solution

Each sample is quickly mixed, allowed to stand for 120 seconds and then immediately subjected to viscosity measurement using a suitable viscometer/rheometer. If the viscosity difference D-B is at least 10% higher than the viscosity difference C-A, then the candidate agent is deemed a DNA gelling agent at the used concentration.

The liquid metering component is preferably capable of providing at least one pre-determined volume of liquid sample in the range of 1 microL -5 mL, preferably in the range of 10 microL-1 mL, and even more preferably in the range of 50 microL - 0.5 mL.

In some preferred embodiments of the invention, the liquid metering component forms part of the type B microfluidic device.

An example of such a liquid metering component can e.g. be found in WO02089670 (A1).

In some preferred embodiments of the invention, the pre-determined volume of liquid which can be provided by the liquid metering component is sufficiently small to avoid complete filling of the first or second micro conduit section while liquid sample simultaneously enters the first or second micro conduit sections, i.e. during their competition for the liquid sample.

For example, the pre-determined volume of liquid which can be provided by the liquid metering component may be at most 95% of the combined volume of the first or second micro conduit section, preferably at most 75% and even more preferably at most 50%.

The pre-determined volume of liquid which can be provided by the liquid metering component may e.g. be at most 95% of the volume of the second micro conduit section, preferably at most 75% and even more preferably at most 50%.

The present inventor has discovered that flow restricting structures (e.g. micro-pillars, constriction, a bead slurry, and/or) improves the possibility of distinguishing between the non-newtonian DNA gels that e.g. are formed in milk samples containing less than 1 mio. Somatic cells per mL.

Thus, in some preferred embodiments of the invention, the first micro conduit section and/or first micro conduit sections contains one or more flow restricting structures.

The one or more flow restricting structures may for example be selected from the group consisting of a one micro pillar or a plurality of micro pillars, a channel constriction, a bead slurry, a polymer sieve, fabric or a combination thereof.

A channel constriction may e.g. have a reduction in its cross-sectional area of at least 10% relative to the cross-sectional area of the adjoining, upstream channel, such as a reduction of at least 30%, or even at least 50%, such as at least 80% or at least 90%.

In some preferred embodiments of the invention, the first conduit section comprises a subsection comprising a plurality of micro pillars, which subsection comprises as least some of the DNA gelling system, such as e.g. at least some of the DNA gelling agent.

Micro pillars are micro protrusions which are located in a micro conduit component, e.g. in a micro channel or a micro chamber. Micro pillars may reach all the way through a micro conduit component or they may have end portions that contact the fluid of the micro conduit components.

A micro pillar may e.g. have a cylindrical, conical, or pyramid-like shape. The typical dimension of a micro pillar is a diameter in the range of 10-600 micron, preferably in the range of 50-500 micron, or even more preferred in the range of 70-300 micron.

The present inventor has observed that the use of micro pillars offers several advantages.

During production of the microfluidic device, the micro pillars act as extra support for dissolved reagents (e.g. a dissolved DNA gelling system) that is spotted into the micro conduit system and subsequently dried.

During use of the microfluidic device, the micro pillars provide the micro conduit system with an increased surface area and dried reagents in the micro conduit system are more efficiently brought into contact with a liquid sample than if no micro pillars were present.

Finally, the micro pillars may serve as flow restriction components and are particularly useful for capturing DNA gel formed by contacting packaged DNA and a DNA gelling agent. The present inventor has found that the micro pillars are capable of capturing the DNA gel without clogging the chamber or channel in which they are placed and they are therefore well-suited for use in micro conduit systems of type B microfluidic devices or other microfluidic devices wherein the non-newtonian viscosity caused by gel-forming polymers has to be converted to a change in flow rate or a change in flow restriction.

In some preferred embodiments of the invention, a means for inspecting the presence or non-presence of liquid is located in first micro conduit section:
- downstream of the subsection comprising a plurality of micro pillars, and/or
- in the downstream part of the subsection comprising a plurality of micro pillars.

A means for inspecting the presence or non-presence of liquid may also be located in the second micro conduit section downstream of a channel constriction.

In some preferred embodiments of the invention, both the first micro conduit section and the second micro conduit section contain a channel constriction.

In some preferred embodiments of the invention, the means for inspecting the presence or non-presence of liquid is one or more optical windows, and preferably one or more optical windows which allow for visual inspection of presence or non-presence of liquid, such as e.g. the liquid sample.

At least some of the means for inspecting are typically located downstream relative to the location in the first micro conduit section where liquid sample contacts the agent capable of providing a change of viscosity in a liquid sample containing a target entity, preferably with the agent being a DNA gelling system.

In some preferred embodiments of the invention, the micro conduit system is designed so as to at least reduce, and preferably avoid, backflow from the first micro conduit section and/or the first micro conduit section.

In some preferred embodiments of the invention, the micro conduit system comprises one or more backflow-reducing structures.

A backflow-reducing structure may for example be an extended liquid communication path between the first and the second micro conduit sections extending into an open, lid-free sample reception structure. Alternatively or additionally, a backflow-reducing structure may be a substantially hydrophobic area in the sample reception structure so that the liquid communication between the first and the second micro conduit sections is more likely to break.

The present inventor has noted that the test results provided by the type B microfluidic device typically are stable for at least 30 minutes, and even more preferably for at least 1 hour. This is advantageous as the timing of the reading of the test results becomes less critical than in conventional test such as the California mastitis test or tests based on direct color change such as the PortaSCC-test provided by Portacheck.

However, the result is preferably available after at most 10 minutes, preferably at most 5 minutes, and even more preferably at most 2 minutes.

In some preferred embodiments of the invention, the agent capable of providing a change of viscosity in a liquid sample containing a target entity is a DNA gelling agent. As described above, DNA gelling agents are for example useful when the target entity is DNA-containing cells or supercoiled DNA in solution.

In other preferred embodiments of the invention, the agent capable of providing a change of viscosity in a liquid sample containing a target entity is an agglutination reagent. The agglutination reagent is for example useful when the target entity is or comprises one or more epitopes. Useful examples of agglutination reagents are e.g. found in WO2008083687 (A1).

In yet other preferred embodiments of the invention, the agent capable of providing a change of viscosity in a liquid sample containing a target entity is a coagulation reagent. A coagulation reagent is for example useful for measuring blood coagulation in which case the target entity is red blood cells. Useful examples of coagulation reagents are e.g. found in WO2008083687 (A1).

The agent capable of providing a change of viscosity in a liquid sample containing a target entity may e.g. be present in the microfluidic device in liquid dissolved form or in dried form. A dried agent is presently preferred.

A dry or dried agent capable of providing a change of viscosity in a liquid sample containing a target entity preferably contains at most 10%(w/w) water, preferably at most 6% (w/w) water, and even more preferably at most 4% (w/w) water.

In the following, a number of preferred exemplary embodiments of the type B microfluidic device are described.

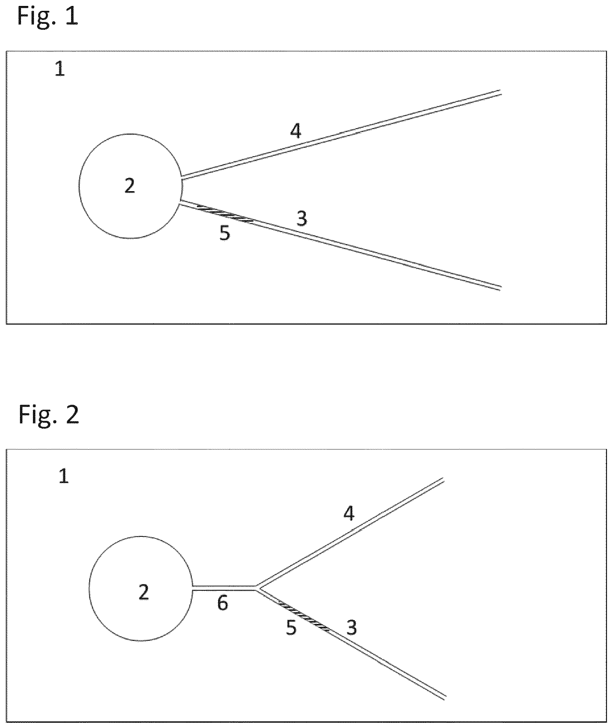

Fig. 1 contains a schematic illustration (top view) of an exemplary embodiment of a type B microfluidic device (1) containing a sample reception structure (2) in direct fluid communication with a first micro conduit section (3) and a second micro conduit section (4). The first micro conduit section (3) contains a DNA gelling system (5) dried on the walls of the part of the first micro conduit section that is closest to the sample reception structure. The lid covering the micro conduit system is not shown.

Fig. 2 is a schematic illustration of another exemplary embodiment of a type B microfluidic device (1) containing a sample reception structure (2) in direct fluid communication with a channel (6) that feeds both the first micro conduit section (3) and a second micro conduit section (4). The lid covering the micro conduit system is not shown.

Fig. 3 is a schematic illustration of yet another exemplary embodiment of a type B microfluidic device (1) containing a sample reception structure (2) in direct fluid communication with a first micro conduit section (3) and a second micro conduit section (4). Again, the first micro conduit section (3) contains a DNA gelling system (5) in dry form. The micro conduit system is covered by a non-transparent lid (7), which lid contains some optical windows (8a, 8b, 8c, 8r₁, and 8r₂). The optical windows 8a, 8b, and 8c, are located over the first micro conduit section downstream of the DNA gelling agent and allows for detecting whether liquid is present or non-present in each window. The higher number of DNA containing cells that the liquid sample contains, the higher viscosity is generated in the first portion of the liquid sample when contacting the DNA gelling system and the less liquid sample enters the first micro conduit section. For very high cell numbers none of the 3 windows will be filled with liquid. For high cell numbers 8a will be filled but 8b and 8c will not be filled. For medium cell numbers 8a and 8b will be filled but 8c will not be filled. For low cell numbers 8a, 8b and 8b will all be filled. 8r₁ and 8r₂ are windows which allow for inspection of the second micro conduit section (the reference). The window 8r₁ is used to ensure that sufficient liquid sample has been used for the test and should be liquidfilled by the end of the test. The window 8r₂ is used to ensure that the microfluidic device has not been overfilled with liquid sample and should be empty by the end of the test.

A type B microfluidic device having the same functionality as the microfluidic device of Fig. 3 could be produced by providing a transparent lid and attaching a non-transparent layer to the lid, non-said non-transparent layer containing openings which define the windows. The non-transparent layer may for example be a non-transparent, adhesive sheet wherein the appropriate windows are of a substantially translucent nature, such as e.g. clear or semi-clear polymer. Alternatively, the non-transparent layer may be a non-transparent coating which has been applied so that non-coated windows are left in the transparent lid.

Fig. 4 is a schematic illustration of yet another exemplary embodiment of a type B microfluidic device (1) which is similar to the device of Fig. 3, but which has a transparent lid (7) and a scale (9) for estimating the cell number of the liquid sample.

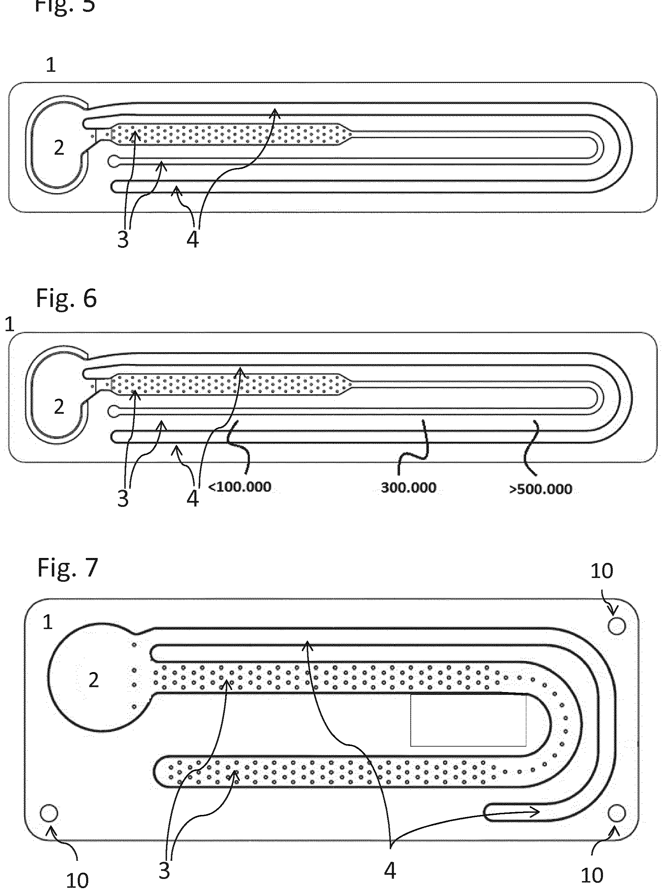

Fig. 5 shows a top view of another exemplary embodiment of a type B microfluidic device (1) containing a sample reception structure (2) and a micro conduit system comprising a first micro conduit section (3) used as measurement-channel and a second micro conduit section (4) used as reference-channel. The circular structures shown in the first micro conduit section are micro pillars protruding from the bottom of the channel.

Fig. 6 shows a top view of the yet another type B microfluidic device (1) of Fig. 5 marking approximately where the liquid front of the liquid sample would stop as a function of the cell number of the sample (cells/mL).

Fig. 7 shows a top view of another exemplary embodiment of a type B microfluidic device (1) wherein DNA-gel-capturing micro pillars have been positioned along the entire length of the first micro conduit section. This microfluidic device furthermore comprises positioning-structures (10) (three circular structures, positioned in three corners). Said positioning-structures allows for fixing the microfluidic device at a precise location, so that e.g. the filling of reagents during production can be performed with high precision or so that the microfluidic device may be positioned accurately in relation to e.g. optical sensors in an automated reading-device.

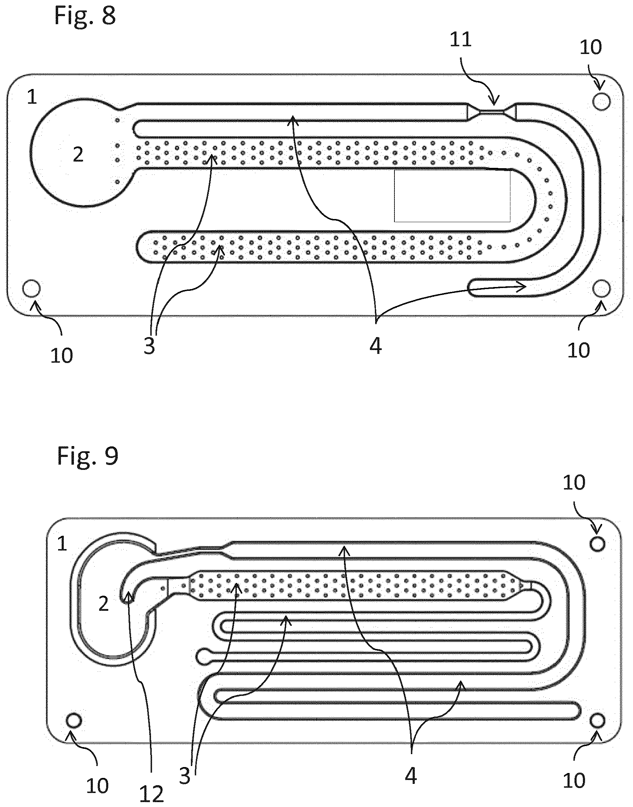

Fig. 8 shows a top view of yet another exemplary embodiment of a type B microfluidic device (1) which resembles that of Fig. 7 but which furthermore contains a channel constriction (11) in the second micro conduit section. The use of a channel constriction or other flow restricting structures in the second micro conduit section has been found to be advantageous as it makes the flow of liquid sample into the second micro conduit section (the reference) better correlated with variations in the basic viscosity of the liquid sample.

Fig. 9 shows a top view of another exemplary embodiment of a type B microfluidic device (1) comprising a backflow-reducing structure (12). In this case, the backflow-reducing structure aids in separating the first micro conduit section and the second micro conduit section when the sample reception structure (2) has been emptied, thus the two separate conduit sections will not be able to draw liquid from one another.

Yet an aspect of the invention pertains to the use of the type B microfluidic device or the system comprising the type B microfluidic device and a liquid metering component for determining the level of somatic cells in a raw milk sample, in which case the agent capable of providing a change of viscosity in a liquid sample containing a target entity is a DNA gelling system and preferably an anionic surfactant.

A further aspect of the invention pertains to a method of determining the level of a target entity in a liquid sample, the method comprising the following steps:
i) providing a system or a type B microfluidic device as defined herein,
ii) applying a volume, and preferably a pre-determined volume, of liquid sample to the sample reception structure,
iii) providing the liquid sample access to flow into the first and second micro conduit sections at the same time, thereby allowing the two sections to compete for the liquid sample, and
iv) detecting the presence or non-presence of liquid in at least one position in at least one of the first and second micro conduit sections, and preferably at least one position in each of the first and second micro conduit sections,
v) determining the level of target entity in the liquid sample based on the detection(s) of step iv).

The method employs the change of viscosity caused by the interaction between a target entity and the agent capable of providing a change of viscosity. A non limiting example is the increase in viscosity which is observed when somatic cells are contacted by a DNA gelling system as defined herein. The level of viscosity change is preferably correlated to the level of target entity present in the liquid sample.

Determining the level of target entity in the liquid sample may be performed the same ways as determining the level of DNA-containing biological cells of the biological source as described above, particularly in the embodiments where the biological source is used directly as the liquid sample.

In particularly preferred embodiments of the invention, the method of determining the level of a target entity in a liquid sample is a method of determining level of somatic cells in raw milk sample, the method comprising the following steps:
i) providing a system or a type B microfluidic device as defined herein, wherein the agent capable of providing a change of viscosity in a liquid sample containing a target entity is a DNA gelling system, and preferably an anionic surfactant.,
ii) applying a volume, and preferably a pre-determined volume, of liquid sample to the sample reception structure, which liquid sample is a raw milk sample,
iii) providing the liquid sample access to flow into the first and second micro conduit sections at the same time, thereby allowing the two sections to compete for the liquid sample, and
iv) detecting the presence or non-presence of liquid in at least two positions in the first micro conduit section, and in at least one position in the second micro conduit section,
v) determining the level of somatic cells in in the liquid sample based on the detection(s) of step iv).

In other preferred embodiments, the method of determining the level of somatic cells in raw milk sample may comprise the following steps:
i) providing a system or a type B microfluidic device as defined herein, wherein the agent capable of providing a change of viscosity in a liquid sample containing a target entity is a DNA gelling system, and preferably an anionic surfactant,
ii) applying a volume, and preferably a pre-determined volume, of liquid sample to the sample reception structure, which liquid sample is a raw milk sample,
iii) providing the liquid sample access to flow into the first and second micro conduit sections at the same time, thereby allowing the two sections to compete for the liquid sample, and
iv) detecting the presence or non-presence of liquid in at least three positions in the first micro conduit section, and in at least two positions in the second micro conduit section,
v) determining the level of somatic cells in in the liquid sample based on the detection(s) of step iv).

It is particularly preferred that step iv) of the method involves detecting the presence or non-presence of liquid in second micro conduit section at at least one position, and wherein the detection(s) relating to the second micro conduit section are used for determining whether the volume of the liquid sample provided to the sample reception structure was too large or too small.

The present invention has been described above with reference to various embodiments. However, other embodiments than the above described are equally possible within the scope of the invention. The different features and steps of various embodiments and aspects of the invention may be combined in other combinations than those described herein.

### EXAMPLES

### Example 1 - Fabrication of a microfluidic device according to the invention

A type B microfluidic device containing a micro conduit system (similar to Fig. 1) was designed and fabricated using CAD/CAM tooling followed by hot-embossing. The micro conduit system was hot-embossed in a 1 mm thick PETG sheet. The rhombic structure, including the vertical micro pillar-structure, was filled with SDS (20% w/w concentration) and was subsequently placed in a desiccator and left to dry, so that the SDS-salt was left as a layer in the structure and around the pillars. After drying a lid was applied to the micro conduit system. The rhombic chamber structure had a width of approx. 2000 micron, a depth of 250 micron and contained 16 micro pillars having a diameter of 150 micron. The inlet channel and outlet channels had a width of 750 micron and a depth of 250 micron.

The lid consisted of a translucent PET adhesive tape. The lid was applied so that it covered the micro conduit system, but left the sample reception structure uncovered. The opposing end of the thus hermetically closed microfluidic structure, was punctured with a pointed tool so that filling by means of capillary action would not be hindered by air-pressure build-up. The device was subsequently tested as described in Example 2.

### Example 2 - Test of the microfluidic device

The microfluidic device of Example 1 was loaded with raw milk that had been milked within the last 12 hours and that had neither been homogenized nor sterilized.

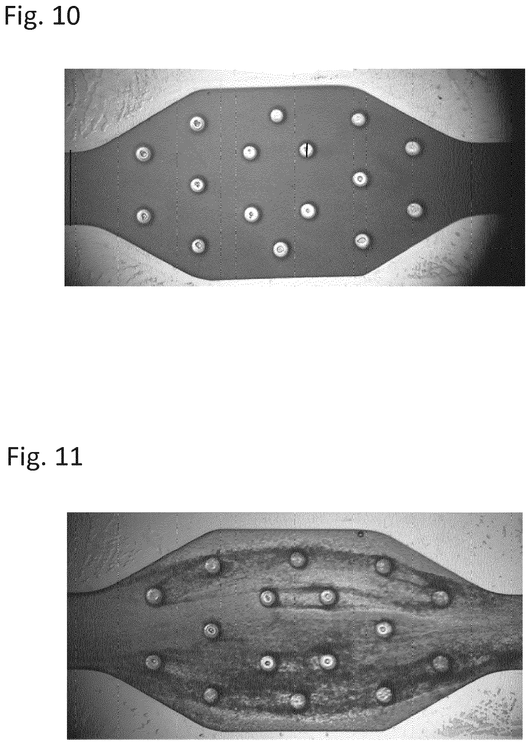

In the first instance - milk stemming from a cow with a relatively low somatic cell count - SCC (<50.000/ml) is chosen and applied to the sample reception structure. The milk was - by means of capillary forces - drawn into the micro conduit system where it contacted the dried SDS. The SDS started to dissolve and mix with the milk and lyse the somatic cells. Very little gel (SDS-histone -complex) was formed, and the milk passed the pillars unhindered and continued to propagate through the micro conduit section downstream of the rhombic structure containing the vertical micro pillars. The lack of gel around the pillars is depicted in fig. 10.

The process described above was repeated with milk having a cell-count of around 300.000 cells/ml (a cell-count of 300.000 indicates the initial onset of mastitis - udder inflammation), which was applied to the sample reception structure of the microfluidic device. When the milk entered the first micro conduit section and contacted the dried SDS, the somatic cells were lysed and DNA-gel was formed. The DNA-gel got trapped and accumulated around the micro pillars (see Fig. 11) and the flow of milk through the first micro conduit section was slowed down. The reduction in flowrate was found to be correlated with the amount of DNA-gel formed which, in turn, ultimately correlated with the number of cells that was originally present in the raw milk.

## Claims

1. A method of determining the level of DNA-containing, biological cells of a biological source, said biological source is a raw milk sample, the method comprising the steps of:
a) providing a microfluidic device comprising:
- a sample reception structure,
- a micro conduit system comprising a DNA gelling system comprising a DNA gelling agent, said DNA gelling system is present in dry form,
b) adding a liquid sample of the biological source to the sample reception structure,
c) contacting the DNA gelling system with a first portion of the sample, thereby generating DNA gel, and
d) measuring a parameter correlated with the viscosity of the first portion of the sample during and/or after contacting the first portion with the DNA gelling system,
e) optionally, measuring a parameter correlated with the viscosity of a portion of the sample which has not been in contact with the DNA gelling system, and
f) determining the level of DNA-containing, biological cells of the biological source using the measurements of step d) and optionally also e), and
wherein
- the method includes step e) and uses the measured parameter of step e) in the determination of step f) and/or
- the measured parameter of step d) at least partially compensates for variation in the viscosity of the liquid sample,
wherein the parameter correlated with the viscosity of the first portion of the sample is one of the following:
i) the flow rate of the first portion of the sample during and/or after contact with the DNA gelling system,
ii) the steady-state position of a liquid in the microfluidic device, which liquid is or has been in liquid contact with the first portion of the sample during and/or after contact with the DNA gelling system,
iii) presence or non-presence of liquid at a specific location in the microfluidic device, which liquid is or has been in liquid contact with the first portion of the sample during and/or after contact with the DNA gelling system, and
iv) an optical property of the first portion.

2. The method according to claim 1, wherein the microfluidic device is a device according to claim 14 or a device provided by the system according to claim 15.

3. The method according to any of the preceding claims, wherein the DNA gelling agent comprises, or even essentially consists of, an anionic or a cationic surfactant, and is preferably an alkyl sulphate salt.

4. The method according to any of the preceding claims, wherein the DNA gelling agent is also capable of lysing eukaryote and/or prokaryote cells.

5. The method according to any of the preceding claims, wherein the DNA gelling system furthermore comprises a cell lysing agent which is different from the DNA gelling agent, and is e.g. a surfactant.

6. The method according to any of the preceding claims, wherein measuring a parameter correlated with the viscosity of a portion of the sample which has not been subjected to DNA gelling involves measuring the parameter on the first portion of the liquid sample before it contacts the DNA gelling agent of the DNA gelling system.

7. The method according to any of the preceding claims, wherein measuring a parameter correlated with the viscosity of a portion of the sample which has not been in contact with the DNA gelling system involves measuring the parameter on a portion of the sample that is different from the first portion.

8. The method according to any of the preceding claims, wherein the parameter correlated with the viscosity measured in step e) is a parameter of the same type as that measured in step d).

9. The method according to any of the preceding claims, wherein determining the level of DNA-containing biological cells of the biological source involves at least a binary determination of whether the level of DNA-containing biological cells is:
- above, or
- at or below
a predefined threshold.

10. The method according to any of the preceding claims, wherein determining the level of DNA-containing biological cells of the biological source involves determination of whether the level of DNA-containing biological cells is within at least 3 different ranges.

11. The method according to any of the preceding claims, wherein determining the level of DNA-containing biological cells of the biological source and the measurement is performed by visual inspection of the microfluidic device.

12. The method according to any of the preceding claims, wherein the measurement(s) is(are) performed electrically or optically and wherein the determination of the level of DNA-containing biological cells of the biological source is performed by a microprocessor based on the measurement(s) of step d) and optionally also step e).

13. The method according to any of the preceding claims, wherein the temperature of the sample is adjusted to a specific target temperature prior to step c), which target temperature is in the range of 1-60 degrees C.

14. A type A microfluidic device for determining the level of DNA-containing cells in a biological sample which is a raw milk sample, the microfluidic device comprising:
- a sample reception structure, wherein the sample comprises raw milk
- a micro conduit system comprising:
- a first micro conduit section in fluid communication with the sample reception structure, said micro conduit section comprising a DNA gelling system comprising a DNA gelling agent
- means for measuring a parameter correlated with the viscosity of a portion of the sample which has not been in contact with the DNA gelling agent, said sample comprising raw milk,
- means for measuring a parameter correlated with the viscosity of a portion of the sample which has been in contact with the DNA gelling system, said sample comprising raw milk,.

15. A system for determining a change of viscosity in a liquid sample which is a raw milk sample, the system comprising:
- a type B microfluidic device comprising:
- a sample reception structure, wherein the sample comprises raw milk,
- a micro conduit system comprising:
- an agent capable of providing a change of viscosity in a liquid sample containing a target entity, which agent is a DNA gelling system present in dry form,
- a first micro conduit section, said first microfluidic conduit section containing the agent,
- a second micro conduit section which is substantially free of the agent,
- both the first and second micro conduit section are so arranged that that a liquid sample can enter them simultaneously,
- means for inspecting the presence or non-presence of liquid in at least one position in at least one of the first and second micro conduit sections, and preferably at least one position in in each of the first and second micro conduit sections, and
- a liquid metering component capable of providing a pre-determined volume of a liquid sample, said liquid metering component preferably a) is a pipette or a pipette-type component capable of metering a pre-determined volume of a liquid sample to the sample reception structure, or b) forms part of the microfluidic device.

## Patentansprüche

1. Verfahren zum Bestimmen der Konzentration DNA-haltiger biologischer Zellen einer biologischen Quelle, wobei die biologische Quelle eine Rohmilchprobe ist, wobei das Verfahren die folgenden Schritte umfasst:
a) Bereitstellen einer mikrofluidischen Vorrichtung, umfassend:
- eine Probenaufnahmestruktur,
- ein Mikroleitungssystem, umfassend ein DNA-Geliersystem, das ein DNA-Geliermittel umfasst, wobei das DNA-Geliersystem in trockener Form vorliegt,
b) Zusetzen einer Flüssigkeitsprobe der biologischen Quelle zu der Probenaufnahmestruktur,
c) In-Kontakt-Bringen des DNA-Geliersystems mit einem ersten Teil der Probe, wodurch ein DNA-Gel erzeugt wird, und
d) Messen eines mit der Viskosität des ersten Teils der Probe korrelierten Parameters während und/oder nach dem In-Kontakt-Bringen des ersten Teils mit dem DNA-Geliersystem,
e) wahlweise Messen eines Parameters, der mit der Viskosität eines Teils der Probe, der nicht mit dem DNA-Geliersystem in Kontakt stand, korreliert ist, und
f) Bestimmen der Konzentration DNA-haltiger biologischer Zellen einer biologischen Quelle unter Verwendung der Messungen von Schritt d) und wahlweise auch e), und
wobei
- das Verfahren Schritt e) einschließt und den gemessenen Parameter von Schritt e) bei der Bestimmung von Schritt f) verwendet und/oder
- der gemessene Parameter von Schritt d) die Schwankung der Viskosität der Flüssigkeitsprobe zumindest teilweise kompensiert,
wobei der mit der Viskosität des ersten Teils der Probe korrelierte Parameter einer der folgenden ist:
i) die Fließrate des ersten Teils der Probe während und/oder nach dem Kontakt mit dem DNA-Geliersystem,
ii) die Steady-State-Position einer Flüssigkeit in der mikrofluidischen Vorrichtung, wobei sich die Flüssigkeit während und/oder nach dem Kontakt mit dem DNA-Geliersystem mit dem ersten Teil der Probe im Flüssigkeitskontakt befindet oder befunden hat,
iii) das Vorliegen oder Nicht-Vorliegen einer Flüssigkeit an einer spezifischen Stelle in der mikrofluidischen Vorrichtung, wobei sich die Flüssigkeit während und/oder nach dem Kontakt mit dem DNA-Geliersystem mit dem ersten Teil der Probe im Flüssigkeitskontakt befindet oder befunden hat, und
iv) eine optische Eigenschaft des ersten Teils.

2. Verfahren nach Anspruch 1, wobei die mikrofluidische Vorrichtung eine Vorrichtung nach Anspruch 14 oder eine von dem System nach Anspruch 15 bereitgestellte Vorrichtung ist.

3. Verfahren nach einem der vorstehenden Ansprüche, wobei das DNA-Geliermittel ein anionisches oder ein kationisches Tensid umfasst oder sogar im Wesentlichen daraus besteht und vorzugsweise ein Alkylsulfatsalz ist.

4. Verfahren nach einem der vorstehenden Ansprüche, wobei das DNA-Geliermittel auch eukaryontische und/oder prokaryontische Zellen lysieren kann.

5. Verfahren nach einem der vorstehenden Ansprüche, wobei das DNA-Geliersystem weiter ein Zelllysemittel umfasst, das von dem DNA-Geliermittel verschieden ist, und z. B. ein Tensid ist.

6. Verfahren nach einem der vorstehenden Ansprüche, wobei das Messen eines Parameters, der mit der Viskosität eines Teils der Probe, der keiner DNA-Gelierung unterzogen wurde, korreliert, das Messen des Parameters beim ersten Teil der Flüssigkeitsprobe, bevor sie mit dem DNA-Geliermittel des DNA-Geliersystems in Kontakt tritt, beinhaltet.

7. Verfahren nach einem der vorstehenden Ansprüche, wobei das Messen eines Parameters, der mit der Viskosität eines Teils der Probe, der nicht mit dem DNA-Geliersystem in Kontakt stand, korreliert, das Messen des Parameters bei einem Teil der Probe, der von dem ersten Teil verschieden ist, beinhaltet.

8. Verfahren nach einem der vorstehenden Ansprüche, wobei der Parameter, der mit der in Schritt e) gemessenen Viskosität korreliert, ein Parameter des gleichen Typs wie der in Schritt d) gemessene ist.

9. Verfahren nach einem der vorstehenden Ansprüche, wobei das Bestimmen der Konzentration DNA-haltiger biologischer Zellen einer biologischen Quelle mindestens eine binäre Bestimmung dessen, ob die Konzentration DNA-haltiger biologischer Zellen:
- oberhalb oder
- bei oder unterhalb
eines zuvor definierten Grenzwerts liegt, beinhaltet.

10. Verfahren nach einem der vorstehenden Ansprüche, wobei das Bestimmen der Konzentration DNA-haltiger biologischer Zellen einer biologischen Quelle die Bestimmung dessen, ob die Konzentration DNA-haltiger biologischer Zellen einer biologischen Quelle innerhalb von mindestens 3 unterschiedlichen Bereichen liegt, beinhaltet.

11. Verfahren nach einem der vorstehenden Ansprüche, wobei das Bestimmen der Konzentration DNA-haltiger biologischer Zellen einer biologischen Quelle und die Messungen durch Sichtprüfung der mikrofluidischen Vorrichtung erfolgen.

12. Verfahren nach einem der vorstehenden Ansprüche, wobei die Messung(en) elektrisch oder optisch durchgeführt wird/werden und wobei die Bestimmung der Konzentration DNA-haltiger biologischer Zellen einer biologischen Quelle durch einen Mikroprozessor basierend auf der/den Messung(en) von Schritt d) und wahlweise auch Schritt e) erfolgt.

13. Verfahren nach einem der vorstehenden Ansprüche, wobei die Temperatur der Probe vor Schritt c) auf eine spezifische Zieltemperatur eingestellt wird, wobei die Zieltemperatur im Bereich von 1-60 Grad C liegt.

14. Mikrofluidische Vorrichtung vom Typ A zum Bestimmen der Konzentration DNA-haltiger biologischer Zellen einer biologischen Quelle, die eine Rohmilchprobe ist, wobei die mikrofluidische Vorrichtung Folgendes umfasst:
- eine Probenaufnahmestruktur, wobei die Probe Rohmilch umfasst
- ein Mikroleitungssystem, umfassend:
- einen ersten Mikroleitungsabschnitt in Fluidverbindung mit der Probenaufnahmestruktur, wobei der Mikroleitungsabschnitt ein DNA-Geliersystem umfasst, das ein DNA-Geliermittel umfasst
- Mittel zum Messen eines Parameters, der mit der Viskosität eines Teils der Probe, der nicht mit dem DNA-Geliermittel in Kontakt stand, korreliert, wobei die Probe Rohmilch umfasst,
- Mittel zum Messen eines Parameters, der mit der Viskosität eines Teils der Probe, der mit dem DNA-Geliersystem in Kontakt stand, korreliert, wobei die Probe Rohmilch umfasst.

15. System zum Bestimmen einer Veränderung der Viskosität in einer Flüssigkeitsprobe, die eine Rohmilchprobe ist, wobei das System Folgendes umfasst:
- eine mikrofluidische Vorrichtung vom Typ A, umfassend:
- eine Probenaufnahmestruktur, wobei die Probe Rohmilch umfasst,
- ein Mikroleitungssystem, umfassend:
- ein Mittel, das eine Veränderung der Viskosität in einer eine Zieleinheit enthaltenden Flüssigkeitsprobe bereitstellen kann, wobei das Mittel ein DNA-Geliersystem ist, das in trockener Form vorliegt,
- einen ersten Mikroleitungsabschnitt, wobei der erste Mikroleitungsabschnitt das Mittel enthält,
- einen zweiten Mikroleitungsabschnitt, der im Wesentlichen frei von dem Mittel ist,
- sowohl der erste als auch der zweite Mikroleitungsabschnitt so angeordnet sind, dass eine Flüssigkeitsprobe gleichzeitig in sie eintreten kann,
- Mittel zum Überprüfen des Vorliegens oder Nicht-Vorliegens von Flüssigkeit an mindestens einer Position in mindestens einem der ersten und zweiten Mikroleitungsabschnitte und vorzugsweise an mindestens einer Position in jedem des ersten und des zweiten Mikroleitungsabschnitts, und
- eine Flüssigkeitsmesskomponente, die ein vorgegebenes Volumen einer Flüssigkeitsprobe bereitstellen kann, wobei die Flüssigkeitsmesskomponente vorzugweise a) eine Pipette oder eine Komponente vom Pipettentyp ist, die ein vorgegebenes Volumen einer Flüssigkeitsprobe zu der Probenaufnahmestruktur messen kann, oder b) Teil der mikrofluidischen Vorrichtung ist.

## Revendications

1. Procédé de détermination du niveau de cellules biologiques contenant de l'ADN d'une source biologique, ladite source biologique est un échantillon de lait brut, le procédé comprenant les étapes de :
a) fourniture d'un dispositif microfluidique comprenant :
- une structure de réception d'échantillon,
- un système de micro-conduit comprenant un système de gélification d'ADN comprenant un agent de gélification d'ADN, ledit système de gélification d'ADN est présent sous une forme sèche,
b) ajout d'un échantillon liquide de la source biologique à la structure de réception d'échantillon,
c) mise en contact du système de gélification d'ADN avec une première partie de l'échantillon, générant ainsi le gel d'ADN, et
d) mesure d'un paramètre corrélé à la viscosité de la première partie de l'échantillon pendant et/ou après la mise en contact de la première partie avec un système de gélification d'ADN,
e) facultativement, mesure d'un paramètre corrélé à la viscosité d'une partie de l'échantillon qui n'a pas été en contact avec le système de gélification d'ADN, et
f) détermination du niveau de cellules biologiques contenant de l'ADN de la source biologique en utilisant les mesures de l'étape d) et facultativement en outre e), et dans lequel
- le procédé inclut l'étape e) et utilise le paramètre mesuré de l'étape e) dans la détermination de l'étape f) et/ou
- le paramètre mesuré de l'étape d) compense au moins partiellement la variation de la viscosité de l'échantillon de liquide,
dans lequel le paramètre corrélé à la viscosité de la première partie de l'échantillon est l'un des suivants :
i) le débit de la première partie de l'échantillon durant et/ou après le contact avec le système de gélification d'ADN,
ii) la position à l'état stable d'un liquide dans le dispositif microfluidique, lequel liquide est ou a été en contact liquide avec la première partie de l'échantillon pendant et/ou après le contact avec le système de gélification d'ADN,
iii) la présence ou la non présence de liquide au niveau d'un emplacement spécifique dans le dispositif microfluidique, lequel liquide est ou a été en contact liquide avec la première partie de l'échantillon pendant et/ou après le contact avec le système de gélification d'ADN, et
iv) une propriété optique de la première partie.

2. Procédé selon la revendication 1, dans lequel le dispositif microfluidique est un dispositif selon la revendication 14 ou un dispositif fourni par le système selon la revendication 15.

3. Procédé selon l'une quelconque des revendications précédentes, dans lequel l'agent de gélification d'ADN comprend, voire consiste sensiblement en, un tensioactif anionique ou cationique, et est de préférence un sel d'alkyl sulfate.

4. Procédé selon l'une quelconque des revendications précédentes, dans lequel l'agent de gélification d'ADN est également capable de lyser les cellules eucaryotes et/ou procaryotes.

5. Procédé selon l'une quelconque des revendications précédentes, dans lequel le système de gélification d'ADN comprend en outre un agent de lyse cellulaire qui est différent de l'agent de gélification d'ADN, et est par exemple un tensioactif.

6. Procédé selon l'une quelconque des revendications précédentes, dans lequel la mesure d'un paramètre corrélé à la viscosité d'une partie de l'échantillon qui n'a pas été soumise à une gélification d'ADN implique la mesure du paramètre sur la première partie de l'échantillon liquide avant qu'il ne vienne au contact de l'agent de gélification d'ADN du système de gélification d'ADN.

7. Procédé selon l'une quelconque des revendications précédentes, dans lequel la mesure d'un paramètre corrélé à la viscosité d'une partie de l'échantillon qui n'a pas été au contact du système de gélification d'ADN implique la mesure du paramètre sur une partie de l'échantillon qui est différente de la première partie.

8. Procédé selon l'une quelconque des revendications précédentes, dans lequel le paramètre corrélé à la viscosité mesurée à l'étape e) est un paramètre du même type que celui mesuré à l'étape d).

9. Procédé selon l'une quelconque des revendications précédentes, dans lequel la détermination du niveau de cellules biologiques contenant de l'ADN de la source biologique implique au moins une détermination binaire du fait que le niveau de cellules biologiques contenant de l'ADN est :
- supérieur, ou
- égal ou inférieur
à un seuil prédéfini.

10. Procédé selon l'une quelconque des revendications précédentes, dans lequel la détermination du niveau de cellules biologiques contenant de l'ADN de la source biologique implique la détermination du fait que le niveau de cellules biologiques contenant de l'ADN se trouve à l'intérieur d'au moins 3 plages différentes.

11. Procédé selon l'une quelconque des revendications précédentes, dans lequel la détermination du niveau de cellules biologiques contenant de l'ADN de la source biologique et la mesure sont réalisées par inspection visuelle du dispositif microfluidique.

12. Procédé selon l'une quelconque des revendications précédentes, dans lequel la (les) mesure(s) est (sont) réalisée(s) de manière électrique ou optique et dans lequel la détermination du niveau de cellules biologiques contenant de l'ADN est réalisée par un microprocesseur basé sur la (les) mesure(s) de l'étape d) et facultativement en outre de l'étape e).

13. Procédé selon l'une quelconque des revendications précédentes, dans lequel la température de l'échantillon est ajustée à une température cible spécifique avant l'étape c), laquelle température cible est dans la plage de 1 à 60 °C.

14. Dispositif microfluidique de type A pour déterminer le niveau de cellules contenant de l'ADN dans un échantillon biologique qui est un échantillon de lait brut, le dispositif microfluidique comprenant :
- une structure de réception d'échantillon, dans lequel l'échantillon comprend du lait brut
- un système de micro-conduit comprenant :
- une première section de micro-conduit en communication fluidique avec la structure de réception d'échantillon, ladite section de micro-conduit comprenant un système de gélification d'ADN comprenant un agent de gélification d'ADN
- un moyen de mesure d'un paramètre corrélé à la viscosité d'une partie de l'échantillon qui n'a pas été en contact avec l'agent de gélification d'ADN, ledit échantillon comprenant du lait brut,
- un moyen de mesure d'un paramètre corrélé à la viscosité d'une partie de l'échantillon qui a été en contact avec le système de gélification d'ADN, ledit échantillon comprenant du lait brut.

15. Système de détermination d'une modification de viscosité dans un échantillon liquide qui est un échantillon de lait brut, le système comprenant :
- un dispositif microfluidique de type B comprenant :
- une structure de réception d'échantillon, dans lequel l'échantillon comprend du lait brut,
- un système de micro-conduit comprenant :
- un agent capable de fournir une modification de viscosité dans un échantillon liquide contenant une entité cible, lequel agent est un système de gélification d'ADN présent sous forme sèche,
- une première section de micro-conduit, ladite première section de conduit microfluidique contenant l'agent,
- une seconde section de micro-conduit qui est sensiblement dépourvue de l'agent,
- les première et seconde sections de micro-conduit étant agencées de manière à ce qu'un échantillon de liquide puisse entrer simultanément dans celles-ci,
- un moyen d'inspection de la présence ou de la non présence d'un liquide à au moins une position dans au moins l'une des première et seconde sections de micro-conduit, et de préférence au moins une position dans chacune des première et seconde sections de micro-conduit, et
- un composant de dosage de liquide capable de fournir un volume prédéterminé d'un échantillon de liquide, ledit composant de dosage de liquide de préférence a) est une pipette ou un composant de type pipette capable de doser un volume prédéterminé d'un échantillon de liquide vers la structure de réception de l'échantillon, ou b) fait partie du dispositif microfluidique.
